# EUROPÄISCHE PATENTANMELDUNG

(11) **EP 1 785 127 A2**
(43) Veröffentlichungstag der Anmeldung: **16.05.2007**
(21) Anmeldenummer: 06022467.2
(22) Anmeldetag: 27.10.2006
(51) Int. Cl.: A61K 6/083

(54) **Reduktion der Schrumpkraft bei Zahnfüllungen**

(30) Priorität: 09.11.2005 DE 102005053775
(71) Anmelder: Heraeus Kulzer GmbH, 63450 Hanau (DE)
(72) Erfinder: Grundler, Andreas, Dr., 42349 Wuppertal (DE); Hoffmann, Marcus, Dr., 61250 Usingen (DE)
(74) Vertreter: Kühn, Hans-Christian

(57) **Zusammenfassung**

Ein selbsthärtendes oder dualhärtendes, dünnfließendes Komposits wird eingesetzt zur Herstellung eines dentalen Liners mit Polymerisation in 2 Stufen mit 2 Abbindezeiten und verzögerter Polymerisationscharakteristik, der zum Einsatz im Bereich der Kavitätenwand in einer dünnen Schicht vorgesehen ist.

## Beschreibung

Die Erfindung betrifft die Reduktion der Schrumpfkraft bei Zahnfüllungen.

Lichthärtende Werkstoffe auf Acrylat-/Methacrylatbasis erfahren bei radikalischer Polymerisation aufgrund des sich bei der Polymerisation reduzierenden Molekülabstandes und der damit einhergehenden Dichteerhöhung einen Volumenschrumpf. Dieser kann durch Zugabe von anorganischen Füllstoffen, wie z.B. Dentalgläsern oder pyrogenen Kieselsäuren, deutlich reduziert werden, da sich ein reduzierter Monomeranteil pro Volumeneinheit ergibt und die Füllstoffe während der Polymerisation nicht schrumpfen.

Bei Dentalanwendungen ist der Volumenschrumpf von großer klinischer Bedeutung, da durch die Materialschrumpfung Zugkräfte auf die Kavitätenwand übertragen werden. Bei Überschreitung einer Maximalkraft kann diese Schrumpfkraft im Extremfall zur Ablösung von der Kavitätenwand führen. In den dadurch entstandenen Randspalt können Bakterien und/oder deren saure Stoffwechsälprodukte eindringen und in der Folge Sekundärkaries entstehen.

Betrachtet man den zeitlichen Verlauf der Schrumpfkraft, zeigt sich folgender typischer Befund:

Direkt nach der Polymerisation ergibt sich durch die Volumenschrumpfung ein Initialwert für die Schrumpfkraft, der dann durch Nachpolymerisation innerhalb von ca. 24 h auf einen Maximalwert ansteigt. Anschließend kommt es durch Wasseraufnahme (im Labor bei einer Lagerung in Wasser bzw. im Mund aus dem Speichel) nach einigen Tagen bis Wochen zu einer leichten Volumenexpansion des Komposits; hierdurch können die Spannungskräfte wieder relaxieren und werden auf ein niedrigeres Niveau zurückgeführt.

Hieraus ergibt sich, dass die entscheidende Einflussgröße der maximale Schrumpfspannungswert nach ca. 24 h ist, da dieser die maximale Kraftbelastung des Verbundsystems Komposit/Adhäsiv/Zahn darstellt.

Es hat nicht an Versuchen gefehlt, schrumpfungsarme Dentalmaterialien zur Verfügung zu stellen: DE 199 05 093 A1 empfiehlt den Einsatz via ringöffnende Metathesepolymerisation (ROMP) härtender, bicyclischer Monomere. Laut DE 198 51 038 A1 ist die Schrumpfung durch Zugabe von Acryloylmorpholin, Cumaronharz, Vinylstearat, Polyvinylacetat oder Alkoholtensiden vor der Polymerisation zu bekämpfen. Gemäß US 5,750,590 schrumpfen kationisch polymerisierbare "Oxetane" (Trimethylenoxide) nur in geringem Maß und eignen sich daher ebenfalls für schrumpfreduzierte Dentalmaterialien. US 6,855,197 B2 beschreibt schrumpfreduzierte Füllungswerkstoffe auf Epoxidharzbasis, die nanoskalige anorganische Oxide als Füllstoffe enthalten. Gemäß US 6,709,271 B2 führt die Verwendung einer Füllstoffmischung mit kugelförmigem Füller der Teilchengröße 200-500 nm und Submikron-Füller der Teilchengröße 20-80 nm zu Schrumpf von bis 1,8% nach der Polymerisation.

Der Gegenstand der vorliegenden Anmeldung bezieht sich in erster Linie auf die Schrumpfkraft und deren Reduktion: Neben den oben beispielhaft erörterten werkstofflichen Eigenschaften beeinflussen auch Verarbeitungsparameter die Schrumpfkraft:

### Lichtleistung

In DE 199 13 890 A1 wurde ein Lichthärtegerät mit Pulsbetrieb zur Behebung von Schrumpfungskraftproblemen vorgeschlagen.

### Polymerisationskinetik

Bei identischem Kompositmaterial können geringere Schrumpfkräfte durch eine anfänglich langsamere Polymerisation bei geringerer Lichtleistung und späterer Anhebung der Lichtleistung auf den Maximalwert erzielt werden (Soft-Start Polymerisation). Durch die geringere Lichtleistung zu Beginn bleibt das Komposit-Material länger fließfähig und kann damit Spannungen besser kompensieren und abbauen (J. Esthet. Restor. Dent. (2003) 15, 93 - 104). In US 20050065227 A1 wird vermutet, dass bei Verwendung mulitfunktioneller Photoinitiatoren die frühen Stufen des Schrumpfs stattfinden, solange das Material noch elastisch ist. Das soll schließlich zu geringeren Schrumpfspannungen führen.

### Geometrie der Versorgung

Schrumpfkräfte können durch die Anwendung einer Inkrementaltechnik beim Aufbau der Versorgung minimiert werden (US 6,783,810 B2). Je mehr Schichten allerdings einzeln gehärtet werden müssen, desto mehr Zeit benötigt der behandelnde Zahnarzt.

Aufgabe dieser Erfindung ist die zumindest teilweise Kompensation der durch die Härtung des Füllungskomposits verursachten Schrumpfkraft.
Dies wird erreicht, indem
ein selbsthärtendes oder dualhärtendes (selbst- und gleichzeitig lichthärtendes) dünnfließendes Komposit in der Anwendung als Liner (folgend als neuartiger Liner bezeichnet) zur Verfügung gestellt wir, das zum Einsatz im Bereich der Kavitätenwand in einer dünnen Schicht vorgesehen ist, und das eine verzögerte Polymerisationscharakteristik aufweist.

Dieser neuartiger Liner ist sehr gering mit lichtaktivierbaren Initiatoren und/oder gering mit einem Redox-Initiator-System versehen. Der selbsthärtende neuartige Liner hat eine Abbindezeit von einigen, z.B. 2 bis 10, Minuten, in der eine erste Festigkeit erreicht wird. Die anschließende vollständige Polymerisation verläuft über mehrere, z.B. 1 bis 3 Stunden. Der dualhärtende, neuartige Liner hat bei Lichtaktivierung idealerweise nur eine geringe Aushärtetiefe von < 1 mm und kann initial durch Lichteinwirkung nur oberflächlich angeliert werden¹. Die anschließende vollständige Polymerisation verläuft im selbsthärtenden Modus ebenfalls über mehrere Stunden. ¹Die Härtungstiefe lässt sich durch Zusatz lichtundurchlässiger Komponenten, z.B. Füllstoffe oder Pigmente, einstellen.

Es ergibt sich demnach bei dem dualhärtenden, dünnfließenden, neuartigen Liner zunächst eine gelierende Lichthärtung und gleichzeitig bzw. nachfolgend die vollständige Selbsthärtung.

In der Praxis wird der zu behandelnde Zahn zunächst gemäß der Total-Etch-Technik geätzt und gebondet bzw. mit einem selbstätzenden Adhäsiv behandelt und anschließend der neuartige Liner in dünner Schicht auf der Kavitätenwand aufgetragen.

Anschließend wird, im Falle des dualhärtenden, neuartigen Liners, initial mittels Licht die Oberflächenstruktur fixiert und dann die im neuartigen Liner verbliebene Kavität mit einem klassischen Füllungskomposit gefüllt, welches abschließend ebenfalls mittels Licht ausgehärtet wird.

Durch die verzögerte, langsam verlaufende Polymerisation des neuartigen Liners härtet dieser verzögert aus, kann dadurch länger fließen und somit den Schrumpf bzw. die Schrumpfkraft des final verwendeten Füllungskomposits zumindest teilweise ausgleichen.

Der neuartige Liner weist bevorzugt folgende Komponenten auf:
Monomerkomponente: von **10** Gew% bis **40** Gew%,
Vernetzerkomponente: von **10** Gew% bis **40** Gew%,
Füllstoffkomponente: von **20** Gew% bis **80** Gew%,
Photoinitiator: bis **0,5** Gew%,
Initiatorsystem: von **0,1** Gew% bis **1,2** Gew%.

Als Monomere kommen die auf dem Dentalgebiet üblichen Monomere in Betracht: Beispiele sind radikalisch polymerisierbare monofunktionelle Monomere wie Mono(meth)acrylate, Methyl-, Ethyl-, Butyl-, Benzyl-, Furfuryl- oder Phenyl(meth)acrylat, polyfunktionelle Monomere wie polyfunktionelle Acrylate bzw. Methacrylate, z.B. Bisphenol-A-di(meth)acrylat, Bis-GMA (ein Additionsprodukt aus Methacrylsäure und Bisphenol-A-diglycidylether), UDMA ("Urethandimethacrylat", z.B. ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth)acrylat, Dodecandioldi(meth)acrylat, Hexyldecandioldi(meth)acrylat, Trimethylolpropantri(meth)acrylat, Pentaerythrittetra(meth)acrylat sowie Butandioldi(meth)acrylat.

Bevorzugt sind Bis-GMA, TEDMA (Triethylenglykoldimethacrylat), UDMA (Urethandimethacrylat), TCD-di-HEMA (Bis (methacryloyloxymethyl)tricyclo[5.2.1.0^{2,6}]decan) und TCD-di-HEA (Bis-(acryloyloxymethyl)tricyclo[5.2.1.0^{2,6}]decan).

Vernetzer: Vernetzermonomere sind z.B. 2,2-Bis-4-(3-methacryloxy-2-hydroxypropyl)-phenyl-propan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16 -diyldimethacrylat (UDMA), d.h. das Urethandimethacrylat aus 2 Mol 2-Hydroxyethylmethacrylat (HEMA) und 1 Mol 2-2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B (2,2'-Bis-(4-hydroxyphenyl)-butan), Bisphenol-F (2,2'-Methylendiphenol) oder 4,4'-Dihydroxydiphenyl, sowie Umsetzungsprodukte von 2 Mol HEMA oder 2-Hydroxypropyl(meth)-acrylat mit, insbesondere 1 Mol, bekannter Diisocyanate, wie z.B. Hexamethylendiisocyanat, m-Xylylendüsocyanat oder Toluylendiisocyanat, als Vernetzermonomere geeignet.

Als Füllstoffe kommen neben den Oxiden TiO₂, ZrO₂, Al₂O₃, SiO₂ weitere Metalloxide wie Zinnoxid, Metallsulfate, weitere Oxide der Nebengruppen des Periodensystems, fluoridabgebende Substanzen, pyrogene oder Fällungskieselsäuren, Dentalgläser wie Aluminosilicatgläser oder Fluoroaluminosilicatgläser, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis (SiO₂, ZrO₂ und/oder TiO₂), Metalloxide mit Primärteilchengröße von ca. 40 bis 300 nm, Splitterpolymerisate mit 10-100 µm Teilchengröße (vgl. R. Janda, Kunststoffverbundsysteme, VCH Verlagsgesellschaft, Weinheim, 1990, Seite 225 ff.) oder deren Mischungen in Frage. Zudem können Verstärkungsmittel wie Glasfasern, Polyamid- oder Kohlenstofffasern eingearbeitet werden.

Der Füllstoffgehalt beträgt in der Regel bevorzugt 5 bis 80 Gew.%, besonders 20 bis 80 Gew.%, relativ zur Gesamtmasse des Dentalmaterials.

Des weiteren können die erfindungsgemäßen Dentalmaterialien weitere, bei Dentalmaterialien übliche Stoffe enthalten, z.B. aus den Gruppen der Pigmente, Stabilisatoren, der antimikrobiellen Additive, UV-Absorber, Thixotropiermittel, Katalysatoren.

Solche Additive werden in eher geringen Mengen eingesetzt, insgesamt 0.01 bis 3.0, besonders 0.01 bis 1.0 Gew.% bezogen auf die Gesamtmasse des Dentalmaterials.

Die Aushärtung der Zusammensetzungen kann je nach Art des verwendeten Polymerisationsinitiators durch thermische, photochemische oder redoxinduzierte radikalische Polymerisation erfolgen.
Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie Azobisisobutyroethylester, Azobisisobutyronitril, Azobis-(2-methylpropionamidin)dihydrochlorid, Benzpinakol oder 2,2-Dimethylbenzpinakol.
Bevorzugte Photoinitiatoren sind Benzophenon, Benzoin sowie deren Derivate oder alpha - Diketone oder deren Derivate wie 9,10-Phenanthrenchinon, Diacetyl oder 4,4-Dichlorbenzil. Besonders bevorzugt werden Campherchinon und 2,2-Dimethoxy-2-phenylacetophenon und besonders bevorzugt alpha -Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.
Als Initiatoren für die bei Raumtemperatur durchgeführte Polymerisation werden vorzugsweise Redox-Initiatorkombinationen, wie z.B. Kombinationen von Benzoyl- oder Laurylperoxid mit N,N-Dimethyl-sym.-xylidin oder N,N-Dimethyl-p-toluidin, verwendet.

Geeignete Füllstoffe und Pigmente sind dem Fachmann bekannt und können z.B. sein Al₂O₃, MgO, ZrO₂, TiO₂, Y₂O₃, YF₃, Fe₂O₃, SiO₂, Gold- oder Silberpartikel, wobei TiO₂ bevorzugt ist.

Zur näheren Erläuterung wird in den folgenden Beispielen die Zusammensetzung von zwei Linern gemäß der Erfindung beschrieben:

### Beispiel 1

Durch Photopolymerisation und Redoxpolymerisation auszuhärtender Liner in Form zweier Pasten:

| Paste A | Gew. % |
|---|---|
| Bis-GMA | 35,41 % |
| Triethylenglycoldimethacrylat | 23,61 % |
| silan. Aerosil | 37,44 % |
| Titandioxid | 3,12 % |
| N,N-Bis-(2-hydroxyethyl)-p-toluidin | 0,41 % |
| DL-Campherchinon | 0,01 % |
| | |

| **Paste B** | **Gew. %** |
|---|---|
| Urethandimethacrylat | 37,29 % |
| Triethylenglycoldimethacrylat | 16,13 % |
| silan. Aerosil | 42,56 % |
| Titandioxid | 3,12 % |
| BPO (Paste 50 %) | 0,84 % |
| BHT | 0,06 % |

### Beispiel 2

Durch Redoxpolymerisation auszuhärtender Liner in der Form zweier Pasten:

| Paste A | Gew. % |
|---|---|
| Bis-GMA | 35,41 % |
| Triethylenglycoldimethacrylat | 23,61 % |
| silan. Aerosil | 40,57 % |
| N,N-Bis-(2-hydroxyethyl)-p-toluidin | 0,41 % |
| | |

| **Paste B** | **Gew. %** |
|---|---|
| Urethandimethacrylat | 37,29 % |
| Triethylenglycoldimethacrylat | 16,13 % |
| silan. Aerosil | 45,68 % |
| BPO Paste | 0,84 % |
| BHT | 0,06 % |

## Patentansprüche

1. Verwendung eines selbsthärtenden oder dualhärtenden, dünnfließenden Komposits, das lichtundurchlässige Komponenten aus den Gruppen der Füllstoffe und Pigmente enthält zur Herstellung eines dentalen Liners mit Polymerisation in 2 Stufen mit 2 Abbindezeiten und verzögerter Polymerisationscharakteristik, der zum Einsatz im Bereich der Kavitätenwand in einer dünnen Schicht vorgesehen ist.

2. Verwendung gemäß Anspruch 1, wobei der Liner in der ersten Stufe eine Abbindezeit von 2-10 Minuten, in der eine erste Festigkeit erreicht wird, aufweist.

3. Verwendung gemäß Anspruch 2, wobei der Liner in der zweiten Stufe eine Abbindezeit von 1-3 Stunden zur Endhärtung aufweist.

4. Verwendung gemäß Anspruch 1, wobei der Liner in der ersten Stufe eine gelierende Lichthärtung und gleichzeitig bzw. nachfolgend in der zweiten Stufe eine vollständige Selbsthärtung aufweist.

5. Verwendung gemäß Anspruch 1, wobei die vorgesehene in der ersten Stufen Härtungstiefe < 1 mm beträgt.

6. Verwendung gemäß Anspruch 1 wobei das Komposit die Zusammensetzung
• Monomerkomponente: von **10** Gew% bis **40** Gew%
• Vernetzerkomponente: von **10** Gew% bis **40** Gew%
• Füllstoffkomponente: von **20** Gew% bis **80** Gew%
• Photoinitiator: bis **0,5** Gew%
• Initiatorsystem: von **0,1** Gew% bis **1,2** Gew%
aufweist.
